# FASCICULE DE BREVET EUROPEEN

(11) **EP 0 993 840 B1**
(45) Date de publication et mention de la délivrance du brevet: **22.04.2009**
(21) Numéro de dépôt: 99402512.0
(22) Date de dépôt: 13.10.1999
(51) Int. Cl.: A61N 1/05

(54) **Sonde implantable dans le réseau veineux coronarien pour la stimulation d'une cavité gauche du coeur**
In Koronarvenen implantierbare Leitung zur Stimulation eines linken Herzhohlraumes
Lead implantable in the coronary veins for stimulating a left cavity of the heart

(30) Priorité: 13.10.1998 FR 9812772
(43) Date de publication de la demande: 19.04.2000
(73) Titulaire: ELA MEDICAL (Société anonyme), 92541 Montrouge (FR)
(72) Inventeur: Ollivier, Jean-Francois, 91190 Villiers le Baole (FR); Bessoule, Frédéric, 91600 Savigny sur Orge (FR); D'Hiver, Philippe, 92130 Issy les Moulineaux (FR); Ritter, Philippe, 92290 Chatenay Malabry (FR)
(74) Mandataire: Dupuis-Latour, Dominique

(56) Documents cités:
- EP-A- 0 601 338
- US-A- 5 322 509
- US-A- 5 423 772
- US-A- 5 476 498
- US-A- 5 683 445

## Description

L'invention concerne les sondes de stimulation cardiaque destinées à être implantées dans le réseau coronarien du coeur pour permettre la stimulation d'une cavité gauche par un « dispositif médical implantable actif » tel que défini par la directive 90/385/CEE du 20 juin 1990 du Conseil des communautés européennes, et plus précisément un dispositif tel qu'un stimulateur cardiaque, défibrillateur et/ou cardioverteur, notamment un stimulateur de type « multisite ».

L'invention sera plus particulièrement décrite dans le cas, le plus fréquent, de la stimulation du ventricule gauche, mais cette application n'est pas limitative, la stimulation de l'oreillette gauche étant également possible par le choix d'un autre site de stimulation.

À la différence des cavités droites pour lesquelles il suffit d'implanter des sondes endocavitaires par le réseau veineux périphérique droit, la mise en place de sondes permanentes dans une cavité du coeur gauche impliquerait des risques opératoires importants, par exemple le risque de passage de bulles vers le réseau vasculaire cérébral situé en aval du ventricule gauche. Pour cette raison, on choisit dans ce cas une sonde introduite dans le réseau coronarien, avec une électrode disposée face au ventricule gauche, l'accès à l'entrée du sinus coronaire se faisant via l'oreillette droite.

L'introduction d'une telle sonde, opérée par des voies endocavitaires, est une intervention particulièrement délicate, compte tenu notamment du fait que la position des points de stimulation est très importante dans le cadre d'un stimulateur de type « multisite », les deux points de stimulation ventricule gauche/ventricule droit devant être les plus éloignés possible pour optimiser la resynchronisation de l'ensemble des cavités cardiaques.

Ce type particulier de sonde doit pour cette raison répondre à un certain nombre de critères précis.

En premier lieu, pour que l'extrémité distale de la sonde puisse être aisément guidée vers l'entrée du sinus coronaire et introduite dans celui-ci quelles que soient les différences de morphologie d'un patient à l'autre (dans certaines pathologies, les oreillettes sont parfois très dilatées) et éviter d'appuyer sur la paroi, la sonde doit être relativement rigide afin de pouvoir être guidée aisément vers l'entrée du sinus au moment de l'implantation.

En second lieu, après avoir trouvé l'entrée du sinus coronaire et introduit l'extrémité de la sonde, le chirurgien doit pouvoir se diriger aisément dans le réseau coronarien, ce qui implique une souplesse relative de la sonde. Mais, au même moment, le chirurgien doit pouvoir lors de la progression de la sonde franchir sans trop de difficulté des barrages tels que des valvules et transmettre la poussée en évitant que la sonde ne forme des boucles dans l'oreillette droite, ce qui au contraire plaide pour une certaine rigidité du corps de sonde.

En troisième lieu, une fois atteint la région du site de stimulation, le chirurgien doit pouvoir aisément ajuster la position de la sonde (aujourd'hui, la sonde est très souvent simplement coincée en extrémité de la veine) et, lorsque le site est bon, le maintien en position de la sonde doit pouvoir être assuré à court et long terme quelle que soit la taille de la veine.

Enfin, il est souhaitable de pouvoir ultérieurement extraire la sonde sans endommager les veines du réseau coronaire.

Idéalement, toutes ces fonctions doivent pouvoir être efficaces pour une majorité de chirurgiens, c'est-à-dire que le système doit être suffisamment proche des techniques existantes et des pratiques courantes.

Le US-A-5 683 445 propose une sonde d'un tel type, avec un ensemble constitué d'un corps de sonde comprenant une gaine creuse élastiquement déformable et essentiellement dépourvue d'élément rigide, dont l'extrémité distale supporte au moins une électrode de stimulation, et d'un mandrin amovible apte à être introduit à l'intérieur de la gaine du corps de sonde et mobile en translation à l'intérieur de celle-ci, ce mandrin étant relativement rigide par rapport à la gaine et plastiquement déformable localement. L'extrémité distale de la sonde présente en l'absence de sollicitation deux courbures inscrites dans deux surfaces distinctes, avec une première surface correspondant à une courbure d'orientation définie par une préforme du corps de sonde, et une seconde surface correspondant à une courbure d'appui définie par une forme naturellement coudée de l'extrémité distale de la sonde. Cette sonde n'est cependant pas optimale sur le plan de la stimulation électrique. En effet, le bout hémisphérique portant l'électrode assure, du fait de la symétrie axiale, un placement identique de la surface active par rapport au myocarde quel que soit l'orientation de l'extrémité de sonde. Mais, du fait de sa position en bout, il présente plusieurs inconvénients sérieux, notamment :
- le risque de stimulation parasite de nerfs par le stimulus électrique,
- la faible densité de courant, donc une moindre efficacité physiologique et une importante consommation d'énergie.

L'un des buts de l'invention est de remédier à ces inconvénients, en proposant une sonde du type général enseigné par le US-A-5 683 445 précité, mais qui soit optimisée sur le plan de la stimulation électrique.

À cet effet, selon l'invention, l'extrémité distale de la sonde est une extrémité auto-orientable, l'électrode étant une électrode sectorielle, avec une partie active latérale s'étendant, dans un plan radial, seulement sur un secteur du corps de sonde, cette partie active de l'électrode sectorielle étant très préférentiellement tournée dans une direction sensiblement perpendiculaire au plan formé par le coude de l'extrémité distale du corps de sonde et faisant saillie radialement par rapport à la surface du corps de sonde.

Selon un certain nombre de caractéristiques subsidiaires avantageuses :
- l'extrémité distale du mandrin est une extrémité effilée munie d'une boule à son sommet ;
- le mandrin comporte une âme gainée sur sa partie proximale d'un tube de renfort procurant une rigidité accrue à cette partie proximale, l'âme émergeant du tube de renfort sur la partie distale, qui présente une plus grande flexibilité ;
- l'extrémité distale du corps de sonde comporte un orifice de traversée axiale, obturé en l'absence de sollicitation extérieure ou intérieure, et permettant le passage et la progression au-delà de l'extrémité distale du corps de sonde d'un mandrin d'angioplastie substitué au mandrin initialement introduit dans le corps de sonde ;
- l'extrémité distale du corps de sonde comporte des moyens élastiques d'appui contre la veine, diamétralement opposés à la partie active de l'électrode, notamment des moyens comportant une contre-courbure terminale additionnelle s'étendant sensiblement dans un plan perpendiculaire au plan formé par le coude de l'extrémité distale du corps de sonde et/ou comportant des organes saillants élastiquement déformables interposés entre la paroi externe de la veine et la surface du myocarde.

On va maintenant décrire un exemple de réalisation de l'invention, en référence aux dessins annexés.

La figure 1 montre le coeur et son réseau coronaire veineux dans lequel une sonde est en cours d'introduction.

La figure 2 illustre schématiquement la configuration de la sonde de la figure 1, avec ses degrés de liberté, au voisinage de l'entrée du sinus coronaire.

La figure 3 est une coupe longitudinale de l'extrémité distale de la sonde.

La figure 4 est une vue perspective montrant la forme de la sonde dans l'espace.

La figure 5 est une vue de bout, selon A-A, de la configuration de la figure 4.

La figure 6 est une vue de dessus, selon B-B, de la configuration de la figure 4.

La figure 7 montre le mandrin dans sa région d'extrémité, avec la caractéristique correspondante donnant le moment d'inertie en fonction du point considéré.

La figure 8 est une représentation schématique illustrant la fonction des moyens élastiques d'appui contre la veine.

Les figures 9 à 14 illustrent six exemples de réalisation différents de ces moyens élastiques d'appui.

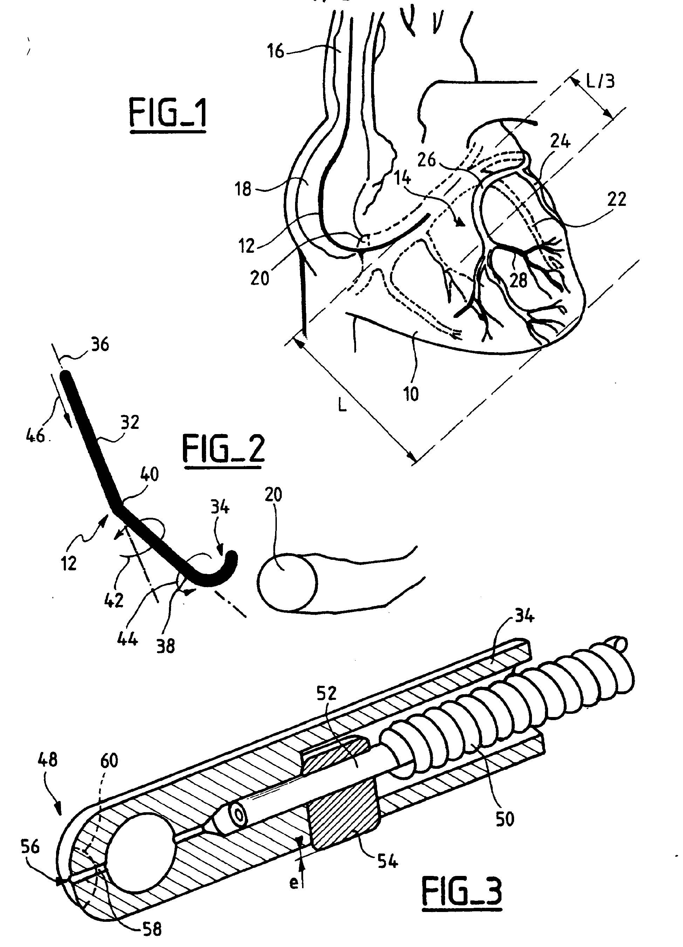

Sur la figure 1, la référence 10 désigne de façon générale le myocarde, dans lequel on a commencé à introduire une sonde 12 destinée, dans cet exemple, à la stimulation du ventricule gauche.

Cette sonde est introduite par voie endocavitaire dans le réseau coronaire veineux, désigné de façon générale par la référence 14, via la veine cave supérieure 16, l'oreillette droite 18 et l'entrée 20 du sinus coronaire veineux. Le réseau coronaire veineux se développe ensuite en plusieurs branches, comprenant les veines cardiaques postéro-latérale 22, latérale 24, la grande veine cardiaque 26 et la veine antéro-latérale 28.

Il s'agit de faire progresser l'extrémité distale de la sonde de manière que cette extrémité, pourvue de l'électrode de stimulation, vienne s'appliquer contre la paroi veineuse, côté coeur, face au ventricule gauche. En pratique, on cherche à atteindre un site de stimulation situé à environ *L*/3 de la veine principale, *L* étant la distance séparant la base de la veine principale de la pointe du ventricule.

Si cette localisation optimale n'est pas respectée, il est nécessaire d'aller beaucoup plus profondément au fond de la veine afin de bien bloquer l'extrémité de sonde en position au site de stimulation. Le site n'étant alors pas optimal, il est nécessaire d'augmenter l'énergie de stimulation pour obtenir l'effet recherché. Pour permettre un guidage aisé de la sonde d'abord vers l'ouverture 20 du sinus coronaire veineux 14, puis à l'intérieur des différentes veines du réseau coronaire, la sonde de l'invention présente la structure générale, connue en tant que telle, illustrée figure 2.

Essentiellement, la sonde 12 comporte un corps de sonde 32 en un matériau souple élastiquement déformable (typiquement un silicone), terminé à son extrémité distale par une partie coudée 34. Au repos, c'est-à-dire en l'absence de sollicitation à la fois intérieure et extérieure du corps de sonde, ce coude 34 est par exemple un coude sur 90° environ, et de rayon 10 à 20 mm. Le corps de sonde est creux, de manière à permettre l'introduction d'un mandrin 36 en un matériau relativement rigide (typiquement un fil d'acier inoxydable MP35N) que l'on peut faire pénétrer plus ou moins dans le corps de sonde de manière à faire varier le rayon de la partie coudée 34. Ainsi, dans la configuration illustrée où l'extrémité distale 38 du mandrin s'arrête juste avant la partie coudée 34, celle-ci conserve sa forme naturelle coudée d'origine ; en revanche, si l'on enfonce plus profondément le mandrin 36 dans le corps de sonde 32, la rigidité du mandrin va redresser le coude 34 et en réduire la courbure.

Par ailleurs, le mandrin peut être déformé à façon par le chirurgien, par exemple sous la forme d'une déformation locale 40 pour permettre une meilleure orientation générale de l'extrémité coudée 34 dans la phase d'approche et faciliter l'accès à l'entrée 20 du sinus coronaire veineux.

On obtient ainsi un système à trois degrés de liberté, à savoir :
- un premier degré de liberté en rotation (flèche 42) autour de la partie la plus proximale du corps de sonde, de manière à permettre une approche globale de l'entrée 20 du sinus par rotation de l'ensemble mandrin 36 + corps de sonde 32,
- un deuxième degré de liberté en rotation (flèche 44) par rotation du corps de sonde autour du mandrin 36, maintenu fixe, pour permettre une approche plus fine, et
- un degré de liberté en translation (flèche 46) par enfoncement du mandrin 36 à l'intérieur du corps de sonde 32, permettant de faire varier la courbure du coude terminal 34.

La simultanéité de ces divers mouvements, ajoutée à la translation d'ensemble de la sonde 12, permet d'entrer aisément dans le sinus coronaire veineux.

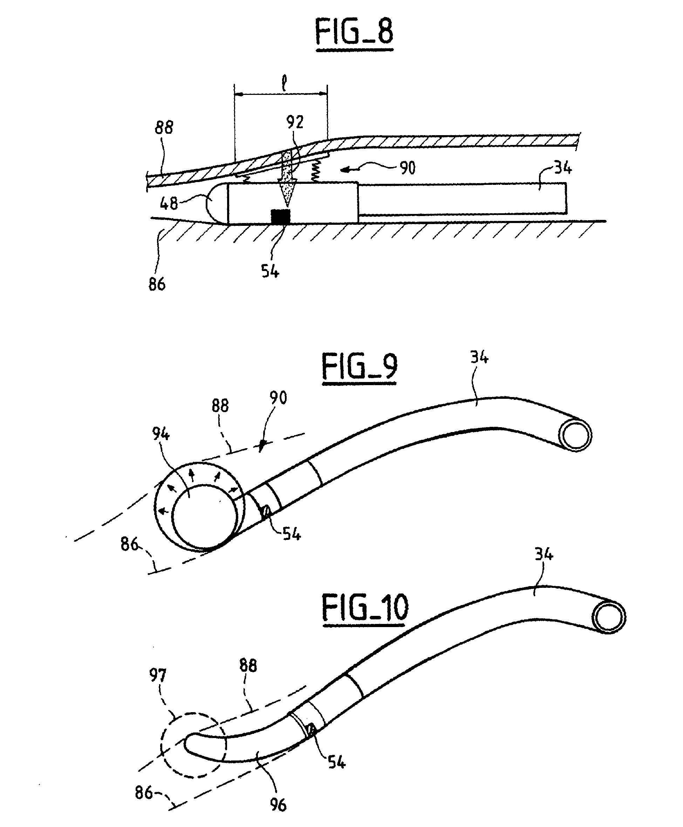

La figure 3 illustre plus précisément la structure de l'extrémité 48 de la sonde. Celle-ci comporte, noyé à l'intérieur du matériau souple du corps de sonde, un conducteur métallique spiralé 50 en contact à son extrémité avec un tube creux 52 en contact avec l'électrode proprement dite 54, qui est, selon l'invention, une électrode de type sectoriel pour les raisons que l'on décrira plus loin en référence aux figures 8 à 14. A son extrémité, la sonde comporte avantageusement une ouverture 56, normalement fermée par la partie de joint 58, mais pouvant être traversée, de façon étanche, par un mandrin d'angioplastie (dont on exposera plus bas le rôle). Pour renforcer l'étanchéité procurée par le joint 58, on peut avantageusement prévoir une cavité frontale 60 permettant de bloquer le sang et constituer ultérieurement par stase un bouchon naturel une fois la sonde définitivement mise en place.

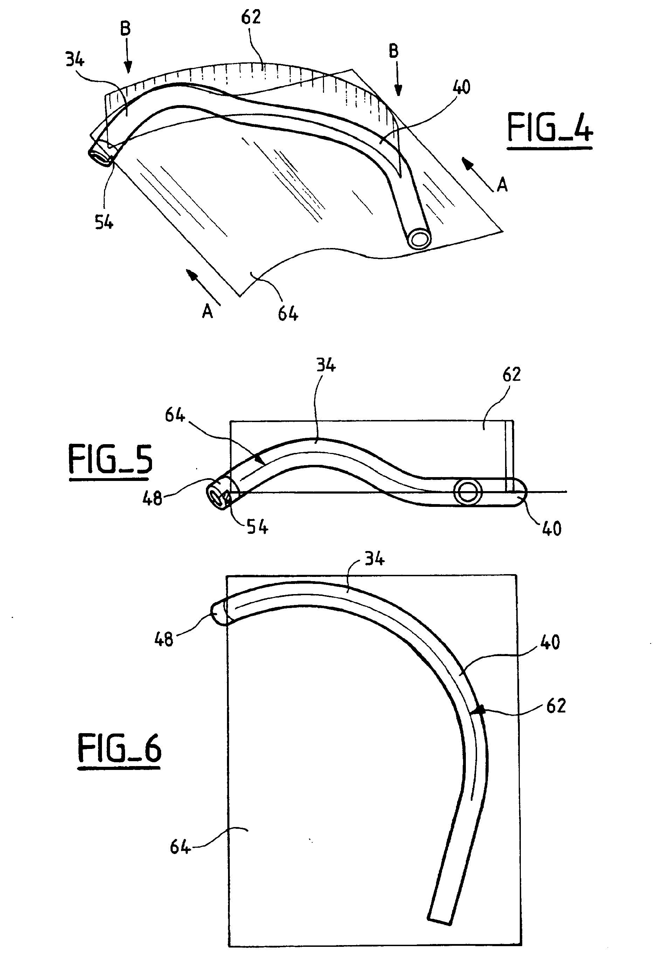

Sur les figures 4 à 6, on a illustré la position dans l'espace de la sonde dans la phase d'approche de l'ouverture du sinus coronaire veineux. La trajectoire du tube est en fait calée sur l'intersection de deux surfaces 62, 64 : la surface 62 correspond à une courbure d'orientation, et elle est définie par la préforme du tube silicone, tandis que la surface 64 correspond à une courbure d'appui résultant de la forme naturellement coudée de l'extrémité distale 34 de la sonde.

Une fois atteinte l'entrée du sinus par la technique précitée, il reste à vaincre de nombreux obstacles.

En effet, lors de sa progression dans les méandres du réseau coronaire veineux, la sonde rencontre des difficultés : frottements, coincements, enchaînements des courbures, resserrements de la veine, passages de valvules, etc. qui nécessitent une poussée de plus en plus forte sur la sonde. Si l'ensemble corps de sonde + mandrin est trop flexible, l'ensemble flambe sous l'effort dans l'oreillette droite, au risque de désengager la sonde du sinus. Pour éviter ce risque, une technique consiste à rentrer de quelques centimètres l'extrémité de la sonde comprenant l'extrémité du mandrin dans le sinus, et faire glisser le corps de sonde sur le mandrin maintenu fixe. On crée ainsi un deuxième appui pour le mandrin, et l'ensemble est alors plus rigide et donc capable de transmettre plus d'effort à l'extrémité de la sonde.

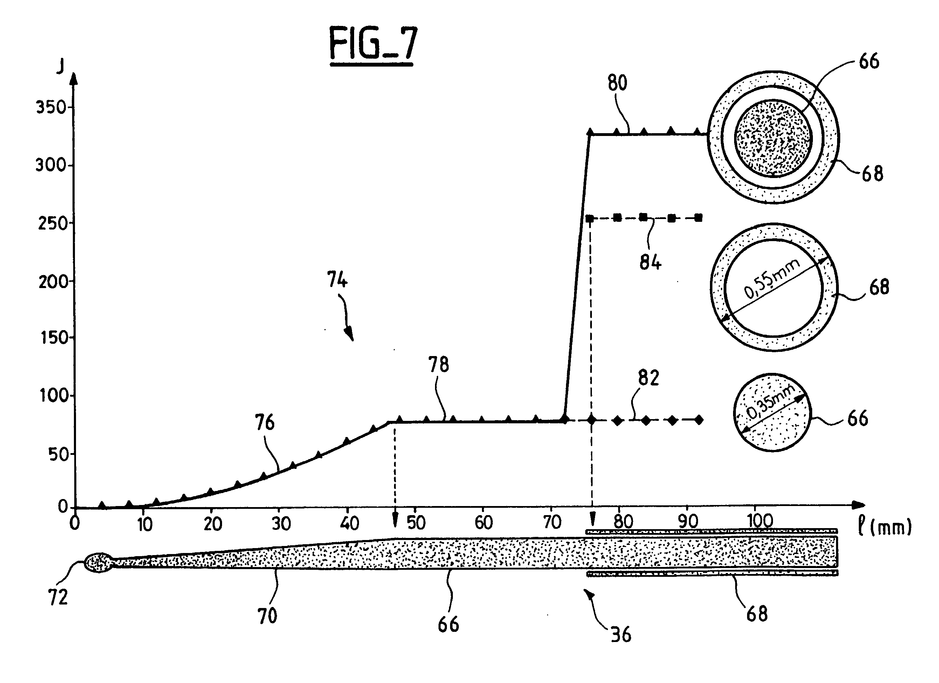

Par ailleurs, pour faciliter l'introduction du mandrin dans la sonde alors que celle-ci est déjà introduite dans le sinus, ce mandrin est avantageusement un mandrin dont l'extrémité distale présente la structure illustrée figure 7, sur laquelle on a également représenté la caractéristique du moment d'inertie correspondant à cette forme de mandrin.

Le mandrin 36 comporte ainsi une partie cylindrique 66 pleine, typiquement de diamètre 0,35 mm, recouverte sur la majeure partie de sa longueur par un tube de renfort 68 gainant la partie centrale 66, le diamètre hors tout de l'ensemble étant typiquement de 0,50 mm.

Côté distal, l'extrémité du mandrin est une extrémité dénudée, dépourvue du tube de renfort 68, sur une longueur totale d'environ 70 mm.

Cette extrémité dénudée se termine par une partie effilée 70, dont la longueur est de l'ordre de 40 mm, elle-même munie à son extrémité d'une boule 72 afin de limiter le frottement sur les spires du conducteur interne 50 (figure 3) du corps de sonde et d'éviter ainsi de « chasser » la sonde et éviter la perforation de la sonde.

Cette structure particulière de mandrin procure une rigidité variable, comme on peut le voir sur la caractéristique 74 donnant le moment d'inertie J en fonction du point considéré du mandrin.

Cette caractéristique comporte une première partie 76 très souple, correspondant à la région effilée 70, permettant d'introduire aisément le mandrin dans le corps de sonde, alors même que celui-ci est déjà introduit dans le sinus.

La région 78 suivante est une région à faible moment d'inertie, donc légèrement moins souple que la précédente, pour permettre l'introduction aisée dans le sinus.

Le moment d'inertie présente ensuite, en direction proximale, une brusque augmentation, dans la région 80 qui correspond à l'addition du moment d'inertie propre 82 de l'âme cylindrique 66 et de celui 84 du tube de renfort 68. Cette rigidité permet de transmettre sans difficulté la poussée, de plus en plus forte, nécessaire à vaincre les divers obstacles rencontrés dans le réseau veineux lors de la progression de la sonde.

Une fois la sonde suffisamment introduite dans le sinus, il peut être avantageux de remplacer le mandrin 36 que l'on vient de décrire par un mandrin d'angioplastie, c'est-à-dire un mandrin très fin comportant une âme métallique surgainée d'un ressort et dont l'extrémité souple peut être introduite directement dans les vaisseaux sans risque de perforation.

Ce mandrin d'angioplastie est introduit dans la cavité centrale du corps de sonde à travers le tube de renfort (évitant ainsi de chasser la sonde), traverse le joint 58 (figure 3) et progresse ensuite, à nu, dans le réseau coronaire, de façon à sélectionner plus aisément une veine collatérale. Une fois la veine sélectionnée, on pourra faire progresser le corps de sonde que l'on glissera sur le mandrin, dont le rôle sera celui d'un simple « passe-fil » de faible diamètre guidant axialement le corps de sonde.

Par ailleurs, du fait de la courbure de l'extrémité coudée 34, en tournant la sonde, il est possible de présenter son extrémité face à l'entrée de la veine cible, et donc d'en faciliter l'accès (du fait que, pendant cette opération, il est souhaitable de disposer d'une sonde hautement flexible afin d'éviter tout coincement intempestif, dans le cas d'une sonde bipolaire il serait avantageux de disposer d'une électrode proximale formée d'un simple ressort en platine iridiée, afin d'éviter d'introduire de la rigidité par un anneau d'électrode extérieur).

De façon caractéristique de l'invention, comme illustré figures 3 et 8, l'électrode 54 est une électrode sectorielle, c'est-à-dire que la région active de cette électrode s'étend dans un plan radial seulement sur un secteur du corps de sonde, et non sur la circonférence de celui-ci (la figure 8 est une représentation dans un plan perpendiculaire au plan P dans lequel s'inscrit la courbure de la partie coudée 34).

Cette configuration permet, dans le cas de la stimulation du ventricule gauche via une veine du réseau coronaire, que l'électrode soit tournée vers l'intérieur du myocarde, ce qui évite la stimulation parasite de nerfs qui, sinon, pourraient être excités par le stimulus électrique.

Par ailleurs, en réduisant la surface active de l'électrode (typiquement une valeur de l'ordre de 2 mm²), on obtient les avantages d'une sonde dite « à haute densité de courant », aussi bien en termes d'efficacité physiologique de la stimulation que de moindre consommation d'énergie.

Si la partie active de l'électrode sectorielle 54 est perpendiculaire au plan formé par le coude 34 de l'électrode (voir par exemple les figures 9 à 14), cette configuration permet un positionnement automatique de la partie active de l'électrode vers l'intérieur du coeur. En effet, cette position est la position de moindre énergie pour le coude du corps de sonde et cette dernière est ainsi « auto-orientée » par de sa géométrie propre.

De plus, l'électrode étant ainsi dirigée vers le myocarde, il est souhaitable de pouvoir exercer sur celle-ci une légère pression, afin d'établir un contact sûr et indépendant des mouvements cardiaques et en éliminant tout risque de flottement dans la veine.

À cet effet, comme illustré schématiquement figure 8, pour améliorer le maintien en place dans la position voulue de l'extrémité 48 de la sonde entre le myocarde 86 et la paroi externe 88 de la veine, on peut prévoir en outre un système élastique 90 exerçant une pression sur la paroi 88 de manière à engendrer par réaction une poussée, schématisée par la flèche 92, qui viendra plaquer l'électrode 54 contre la surface du myocarde 86. De plus, ces moyens élastiques, qui s'étendent de préférence sur une région relativement longue ℓ, permettent de coincer en place, mécaniquement, la tête de sonde en évitant tout retrait ou mouvement intempestif de celle-ci.

Ce système élastique est placé diamétralement opposé à la partie active de l'électrode 54 et peut être réalisé de diverses manières, notamment celles illustrées à titre d'exemple sur les figures 9 à 14. Enfin, et comme on peut le voir notamment sur la figure 3, l'électrode 54 est légèrement saillante (surépaisseur *e*) dans sa région active afin de favoriser le contact avec la zone correspondante du myocarde.

Dans le mode de réalisation de la figure 9, les moyens élastiques 90 comportent une boule 94 située en aval (dans le sens distal) de l'électrode 54. Cette boule permet en particulier un coincement efficace dans toutes les directions et un retrait aisé de la sonde. Elle peut éventuellement être constituée d'un matériau chargé d'un stéroïde à libération progressive, de manière en elle-même classique, le silicone chargé par le stéroïde ayant avantageusement la propriété d'augmenter de volume en place par absorption

Cette sphère peut être éventuellement gonflable et dégonflable depuis l'intérieur du corps de sonde : dans ce cas, une dépression vient écraser la boule 94 pour l'insertion, puis on laisse pénétrer l'air pour que la boule 94 retrouve sa forme sphérique naturelle d'origine ; en variante, cette boule 94 peut être également gonflée *in situ,* notamment avec injection d'un fluide radio-opaque (ce qui présente un avantage par rapport aux tubes du type dit « à pelage »).

Dans le mode de réalisation de la figure 10, les moyens élastiques comprennent un contre-coude 96, en aval de l'électrode 54.

Ce mode de réalisation particulièrement préféré présente de nombreux avantages :
- le contre-coude 96 joue le rôle d'une « corne de pilotage» : l'extrémité de celle-ci étant dirigée dans le sens opposé à la fin de la courbure d'appui, le contre-coude est systématiquement dirigé vers l'ostium des veines secondaires et la translation de l'extrémité de la sonde au voisinage de l'ostium visé permet, dans la plupart des cas, un accès sans difficulté ;
- cette sonde offre une large plage de fonctionnement, avec un débattement important de la courbure d'appui, typiquement de l'ordre de trois fois le diamètre de la veine, garantissant un bon contact électrode/endocarde quelque soit la dimension de la veine ;
- la rétention étant assurée par le coude 34 et le contre-coude 96, il n'est pas indispensable d'introduire la sonde jusqu'à l'extrémité de la veine, ce qui permet une optimisation du choix du site de stimulation et une meilleure dépolarisation d'ensemble des deux ventricules (en maximisant la on obtient une sonde monocorps de faible encombrement, dépourvue de barbes, sphères ou autre système d'accrochage ;
- distance entre les sites de stimulation ventricule gauche/ventricule droit) ;
- le produit est relativement facile à fabriquer, la technique de mise en forme du tube étant comparable à celle d'une sonde auriculaire en J ;
- la sonde est facile à extraire le cas échéant, du fait de l'absence de barbes à retrousser ou de boule à écraser ou dégonfler.

En complément de ce mode de réalisation, il est possible de prévoir une sphère 97 à l'extrémité du contre-coude 96, pour diminuer localement la contrainte sur la paroi de la veine.

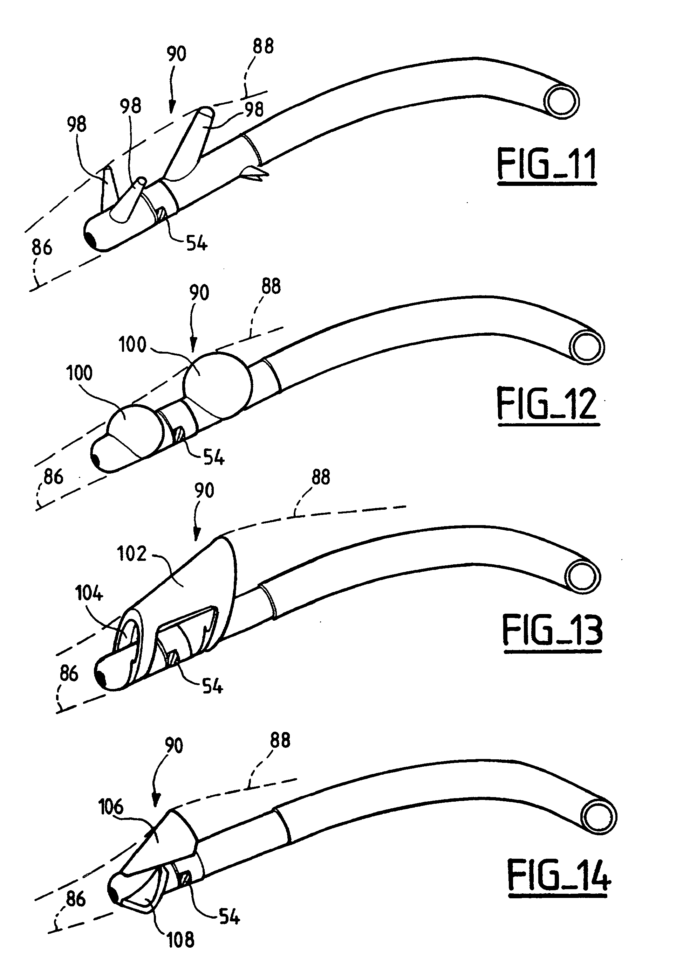

Dans le mode de réalisation de la figure 11, les moyens élastiques 90 comportent une série de barbes 98 située en amont et/ou en aval de l'électrode 54. Il y a lieu de noter que, à la différence des barbes des sondes endocavitaires classiques, la fonction principale de ces barbes 98 est de procurer un appui élastique à la tête de sonde entre la paroi 88 de la veine et le myocarde 86 et non (ou seulement subsidiairement) de rétention mécanique par effet « anti-retour ».

Dans le mode de réalisation de la figure 12, les moyens élastiques 90 comprennent un chapelet de sphères 100 situées à la fois en amont et en aval de l'électrode 54. Les différentes remarques formulées à propos de la boule terminale unique du mode de réalisation de la figure 9 sont applicables à cet autre mode de réalisation.

Dans le mode de réalisation de la figure 13, les moyens élastiques 90 comprennent un toit 102 situé à distance de la tête de sonde et relié à celle-ci par des montants 104 permettant un affaissement du toit contre le corps de sonde pendant la phase d'introduction.

Dans le mode de réalisation de la figure 14, les moyens élastiques 90 comprennent une jupe creuse 106 agissant d'une manière comparable à celle des barbes 98 du mode de réalisation de la figure 11, cette jupe étant combinée à des ailerons 108 pour le guidage de la sonde lors de sa descente dans le réseau coronaire veineux.

## Revendications

1. Un ensemble, implantable dans le réseau veineux coronarien, pour la stimulation d'une cavité gauche du coeur en coopération avec un dispositif médical implantable actif, notamment un stimulateur cardiaque, défibrillateur et/ou cardioverteur, comprenant :
- un corps de sonde (12), comprenant une gaine creuse (32) élastiquement déformable et essentiellement dépourvue d'élément rigide, dont l'extrémité distale (34) supporte au moins une électrode de stimulation (54),
- un mandrin amovible (36), apte à être introduit à l'intérieur de la gaine du corps de sonde et mobile en translation à l'intérieur de celle-ci, ce mandrin étant relativement rigide par rapport à la gaine et plastiquement déformable localement,
et dans lequel l'extrémité distale (34) de la sonde présente en l'absence de sollicitation deux courbures inscrites dans deux surfaces distinctes, avec une première surface (62) correspondant à une courbure d'orientation définie par une préforme du corps de sonde, et une seconde surface (64) correspondant à une courbure d'appui définie par une forme naturellement coudée de l'extrémité distale (34) de la sonde,
**caractérisé en ce que** l'extrémité distale (34) de la sonde est une extrémité auto-orientable, l'électrode étant une électrode sectorielle, avec une partie active latérale (54) s'étendant, dans un plan radial, seulement sur un secteur du corps de sonde.

2. L'ensemble de la revendication 1, dans lequel la partie active de l'électrode sectorielle est tournée dans une direction sensiblement perpendiculaire au plan formé par le coude (64) de l'extrémité distale du corps de sonde.

3. L'ensemble de la revendication 1, dans lequel la partie active de l'électrode fait saillie radialement (e) par rapport à la surface du corps de sonde.

4. Un ensemble selon la revendication 1, dans lequel l'extrémité distale du mandrin est une extrémité effilée (70) munie d'une boule (72) à son sommet.

5. L'ensemble de la revendication 1, dans lequel le mandrin comporte une âme (66) gainée sur sa partie proximale d'un tube de renfort (68) procurant une rigidité accrue à cette partie proximale, l'âme émergeant du tube de renfort sur la partie distale, qui présente une plus grande flexibilité.

6. Un ensemble selon la revendication 1, dans lequel l'extrémité distale du corps de sonde comporte un orifice (58) de traversée axiale, obturé en l'absence de sollicitation extérieure ou intérieure, et permettant le passage et la progression au-delà de l'extrémité distale du corps de sonde d'un mandrin d'angioplastie substitué au mandrin initialement introduit dans le corps de sonde.

7. L'ensemble de la revendication 1, dans lequel l'extrémité distale du corps de sonde comporte des moyens élastiques (90) d'appui contre la veine, diamétralement opposés à la partie active de l'électrode.

8. L'ensemble de la revendication 7, dans lequel les moyens élastiques d'appui comportent une contre-courbure terminale (96) additionnelle, s'étendant sensiblement dans un plan perpendiculaire au plan formé par le coude de l'extrémité distale du corps de sonde.

9. L'ensemble de la revendication 7, dans lequel les moyens élastiques d'appui comportent des organes saillants élastiquement déformables (94 ; 96 ; 98 ; 100 ; 102, 104 ; 106) interposés entre la paroi externe de la veine (88) et la surface (86) du myocarde.

## Claims

1. An apparatus implantable into the coronary venous network for the stimulation of a left cavity of the heart in cooperation with an active implantable medical device, particularly a pacemaker, an defibrillator and/or a cardioverter, comprising :
- a catheter body (12) comprising a hollow sheath (32) that is elastically deformable and essentially free of any rigid component, and the distal end (32) of which supports at least one stimulation electrode (54),
- a guide wire (36) being able to be introduced into the interior of the sheath of the catheter body and translatable inside the latter, said guide wire being relatively rigid compared to the sheath and locally elastically deformable,
and wherein the distal end (34) of the catheter presents in the absence of stress two curvatures inscribed in two distinct surfaces, with a first surface (62) corresponding to an orientation curvature defined by a preform of the catheter body and a second surface (64) corresponding to a supporting curvature defined by a naturally bended form of the distal end (34) of the catheter,
**characterized in that** the distal end (34) of the catheter is a self-orientable end, the electrode being a sector electrode, with a lateral active part (54) extending in a radial plan, only on one sector of the catheter body.

2. Apparatus according to Claim 1, wherein the active part of the sector electrode is directed in a direction essentially perpendicular to the plan formed by the bend (64) of the distal end of the catheter body.

3. Apparatus according to Claim 1, wherein the active part of the electrode radially (e) projects from the surface of the catheter body.

4. Apparatus according to Claim 1, wherein the distal end of the guide wire is a tapered end (70) having a ball (72) at its apex.

5. Apparatus according to Claim 1, wherein the guide wire comprises a core (66) sheathed on its proximal part with a reinforcement tube (68) providing an increased rigidity to such proximal part, wherein the core emerges from the reinforcement tube on the distal part, that is more flexible.

6. Apparatus according to Claim 1, wherein the distal end of the catheter body comprises an axial through-orifice (58) that is closed in the absence of external or internal stress and permits the passage and the progression, beyond the distal end of the catheter body, of an angioplasty guide wire replacing the guide wire initially introduced into the catheter body.

7. Apparatus according to Claim 1, wherein the distal end of the catheter body comprises elastic means (90) pressing against the vein, diametrically opposed to the active part of the electrode.

8. Apparatus according to Claim 7, wherein the pressing elastic means comprise a terminal additional counter-bend (96) extending essentially in a plan perpendicular to the plan formed by the bend of the distal end of the catheter body.

9. Apparatus according Claim 7, wherein the supporting elastic means comprises elastically deformable projecting parts (94 ; 96 ; 98 ; 100 ; 102, 104 ; 106) interposed between the external wall of the vein(88) and the surface (86) of the myocardium.

## Patentansprüche

1. Eine in das Koronarvenennetz implantierbare, medizinische Vorrichtung zur Stimulation eines linken Herzhohlraums in Kooperation mit einer aktiven medizinischen Einrichtung, insbesondere einem Herzschrittmacher, umfassend :
- einen Sondenkörper (12) umfassend eine hohle, verformungselastischen Hülle (32) im wesentlichen ohne steifes Element, deren Distalende (34) wenigstens eine Stimulationselektrode (54) trägt,
- einen abnehmbaren, Führungsdraht (36), der geeignet ist, in den Innenraum der Sondenkörperhülle eingeführt zu werden und innerhalb des letzteren translationsbewegbar ist, wobei dieser Führungsdraht verhältnismässig steif bezüglich die Hülle und örtlich plastisch verformbar ist,
und in welche das Distalende (34) der Sonde bei Abwesenheit von Belastung zwei in zwei verschiedenen Oberflächen eingeschriebenen Krümmungen umfasst, mit einer ersten Oberfläche (62) entsprechend einer durch einen Vorformling des Sondenkörpers bestimmten Ausrichtungskrümmung und einer zweiten Oberfläche (64) entsprechend einer durch eine natürlicherweise gekrümmte Form des Distalendes (34) der Sonde bestimmten Stützkrümmung,
**dadurch gekennzeichnet, dass** das Distalende (34) der Sonde ein selbstausrichtbares Ende ist, wobei die Elektrode eine Sektorelektrode ist mit einem in einem radialen Plan nur auf einem Sektor der Sondenkörper ausdehnenden aktiven Seitenteil.

2. Die Vorrichtung gemäss Anspruch 1, in welcher der aktive Teil der Sektorelektrode in eine wesentlich zum jenem, durch die Krümmung (64) des Distalendes des Sondenkörpers gebildeten Plan senkrechte Richtung gewandt ist.

3. Die Vorrichtung gemäss Anspruch 1, in welcher der aktive Teil der Elektrode radial (e) über die Oberfläche des Sondenkörpers vorsteht.

4. Die Vorrichtung gemäss Anspruch 1, in welcher das Distalende des Führungsdrahts ein sich verjüngendes Ende ist mit einer Kugel (72) an der Spitze.

5. Die Vorrichtung gemäss Anspruch 1, in welcher der Führungsdraht eine auf seinem Proximalteil mit einem Verstärkungsrohr (68) ummantelte Seele (66) umfasst, wobei die Steifigkeit dieses Proximalteils erhöht wird, und wobei die Seele aus dem Verstärkungsrohr auf dem Distalteil austritt, deren Flexibilität grösser ist.

6. Eine Vorrichtung gemäss Anspruch 1, in welcher das Distalende des Sondenkörpers eine Öffnung (58) für eine Axialdurchführung, die in Abwesenheit von Belastung von aussen oder von innen verschlossen ist, umfasst, wobei die Öffnung den Durchlauf und den Fortschritt jenseits des Distalendes des Sondenkörpers eines den anfänglich in den Sondenkörper eingeführtes Führungsdraht ersetzenden Angioplastie-Führungsdrahts erlaubt.

7. Die Vorrichtung gemäss Anspruch 1, in welcher das Distalende des Sondenkörpers elastische, zur Anlage gegen die Venen bestimmte, diametral dem aktiven Teil der Elektrode entgegengesetzte Mittel (90) umfasst.

8. Die Vorrichtung gemäss Anspruch 7, in welcher die zur Anlage bestimmten elastischen Mittel eine zusätzliche End-Gegenkrümmung (96) umfasst, die sich wesentlich in einem zum durch die Krümmung des Distalendes des Sondenkörpers gebildeten Plan senkrechten Plan ausdehnt.

9. Die Vorrichtung gemäss Anspruch 7, in welcher die zur Anlage bestimmten elastischen Mittel vorstehende, elastisch verformbare Glieder (94 ; 96 ; 98 ; 100 ; 102, 104 , 106) umfassen, die zwischen dem Aussenwand der Vene (88) und der Oberfläche (86) des Myokards zwischengelegt sind.
